# EUROPÄISCHE PATENTSCHRIFT

(11) **EP 3 463 568 B1**
(45) Veröffentlichungstag und Bekanntmachung des Hinweises auf die Patenterteilung: **21.04.2021**
(21) Anmeldenummer: 17730036.5
(22) Anmeldetag: 24.05.2017
(51) Int. Cl.: A61N 1/39, A61N 1/08, G01R 27/26, A61N 1/04, A61N 1/372, G01R 31/58

(54) **PRÜFVORRICHTUNG ZUM ÜBERPRÜFEN WENIGSTENS EINER ERSTEN MEDIZINISCHEN ELEKTRODE**
TESTING DEVICE FOR CHECKING AT LEAST ONE FIRST MEDICAL ELECTRODE
DISPOSITIF DE TEST POUR VÉRIFIER AU MOINS UNE PREMIÈRE ÉLECTRODE MÉDICALE

(30) Priorität: 27.05.2016 AT 504792016
(43) Veröffentlichungstag der Anmeldung: 10.04.2019
(73) Patentinhaber: Leonh. Lang, 6020 Innsbruck (AT)
(72) Erfinder: FÖGER, Simon, 6410 Telfs (AT); STÄRZ, Ronald, 6073 Sistrans (AT)
(74) Vertreter: Torggler & Hofinger Patentanwälte
(86) Internationale Anmeldenummer: PCT/AT2017/060138
(87) Internationale Veröffentlichungsnummer: WO 2017/201560

(56) Entgegenhaltungen:
- WO-A1-2015/143460
- US-A1- 2007 093 871
- US-A1- 2012 179 234
- US-A1- 2012 299 607

## Beschreibung

Die Erfindung betrifft eine Prüfvorrichtung zum Überprüfen wenigstens einer medizinischen Elektrode, mit den Merkmalen des Oberbegriffs des Anspruchs 1.

Zu überprüfende Elektroden gehen z. B. aus der US 2015/0045869 A1 hervor. Dort sind zwei gemeinsam konfektionierte medizinisch Elektroden in Form von Defibrillationselektroden gezeigt.

Bisherige Prüfvorrichtungen funktionierten auf rein manueller Basis, indem durch einen Mitarbeiter das Vorliegen eines Kontaktes zwischen Anschlüssen der Elektrode und der eigentlichen Elektrodenfläche (Leitfläche) visuell überprüft wurde und die elektrische Konnektierung mittels eines Multimeters festgestellt wurde. Der Prüfschritt war gesondert zu den wertschöpfenden Vorgängen vorzusehen. Ein solches Vorgehen ist fehleranfällig und aufwändig.

US 2012/0299607 A1, WO 2015/143460 A1 und US 2012/0179234 A1 zeigen Prüfvorrichtungen für Defibrillatorelektroden und US 2007/0093871 A1 zeigt eine Prüfvorrichtung zur Qualitätsprüfung von Elektrodenleitungen.

Aufgabe der Erfindung ist die Bereitstellung einer Prüfvorrichtung, welche ein zumindest teilautomatisiertes Prüfen, welches während eines wertschöpfenden Vorganges stattfinden kann, gestattet.

Diese Aufgabe wird durch eine Prüfvorrichtung mit den Merkmalen des Anspruchs 1 gelöst. Vorteilhafte Ausführungsbeispiele der Erfindung sind in den abhängigen Ansprüchen definiert.

Die erfindungsgemäße Prüfvorrichtung arbeitet ohne die Herstellung einer mechanischen elektrischen Kontaktierung zwischen der bzw. den zu prüfenden medizinischen Elektrode(n) und der bzw. den Messelektroden. Der Prüfvorgang selbst kann vollautomatisch erfolgen, die Bestückung der Prüfvorrichtung kann zumindest teilautomatisch erfolgen.

An sich genügt eine einzige (erste) Messelektrode zur Ausführung des Prüfvorganges. Zur Erhöhung der Sicherheit (insbesondere Sicherheit gegenüber Fehlbedienung und Manipulationssicherheit und Messstabilität der Prüfung) kann die Anordnung zumindest einer zweiten Messelektrode vorgesehen sein. Nur wenn die Auswerteeinheit in Bezug auf die Signale beider sich ergebenden Kapazitäten feststellt, dass die medizinische Elektrode die Prüfung bestanden hat, wird diese frei gegeben. Bei einer solchen Ausführungsform ist vorgesehen, dass
- die Prüfvorrichtung weiters zumindest eine zweite Messelektrode aufweist, die so relativ zu der ersten zu überprüfenden medizinischen Elektrode anordenbar ist, dass die zumindest eine zweite Messelektrode und die erste zu überprüfende medizinische Elektrode eine zweite Kapazität bilden
- die Auswertevorrichtung, dazu ausgebildet ist, aus einem gemessenen Impedanzverlauf einer in Reaktion auf eine durch die Signalerzeugungsvorrichtung erzeugte Wechselspannung aufgrund der zweiten Kapazität hervor gerufenen Impedanz zumindest ein zweites Prüfergebnis in Bezug auf die zweite Kapazität zu bestimmen.

Dabei kann bevorzugt vorgesehen sein, dass die Flächen der zumindest einen ersten und der zumindest einen zweiten Messelektrode unterschiedlich groß sind. Dies gestattet die Verwendung zweier unterschiedlicher Freigabefenster in Bezug auf die eine zu prüfende medizinische Elektrode, auch bei Verwendung nur einer Wechselspannung mit einer Frequenz.

Die erste und die zweite Messelektrode können an sich beliebig in Bezug auf die zu prüfende medizinische Elektrode angeordnet werden (z. B. nebeneinander).

Bevorzugt ist aber zur Erzielung einer kompakten Anordnung vorgesehen, dass die zumindest eine erste Messelektrode als Kreisring ausgebildet ist und die zumindest eine zweite Messelektrode innerhalb des Kreisrings angeordnet und durch einen Isolator vom Kreisring elektrisch getrennt ist.

Bevorzugt ist vorgesehen, dass die Prüfvorrichtung zur gemeinsamen oder gleichzeitigen Überprüfung wenigstens einer ersten medizinischen Elektrode und einer zweiten medizinischen Elektrode ausgebildet ist. Z. B. weisen Defibrillationselektroden ein Elektrodenpaar auf, andere medizinische Elektroden können in kleinerer (d. h. in Isolation) oder in größerer Anzahl zu einer funktionellen Einheit zusammengefasst sein. Bei dieser Ausführungsform weist die Prüfvorrichtung weiters auf:
- zumindest eine dritte Messelektrode, die so relativ zu der zweiten zu überprüfenden medizinischen Elektrode anordenbar ist, dass die zumindest eine dritte Messelektrode und die zweite zu überprüfende medizinische Elektrode eine dritte Kapazität bilden, wobei
- die Signalerzeugungsvorrichtung dazu ausgebildet ist, über eine Wechselspannung zu erzeugen, mittels welcher die dritte Kapazität beaufschlagbar ist
- die Auswertevorrichtung, dazu ausgebildet ist, aus einem gemessenen Impedanzverlauf einer in Reaktion auf die durch die Signalerzeugungsvorrichtung erzeugte Wechselspannung aufgrund der dritten Kapazität hervor gerufenen Impedanz zumindest ein erstes Prüfergebnis in Bezug auf die dritte Kapazität zu bestimmen.

Natürlich kann die gemeinsame oder gleichzeitige Überprüfung derart erfolgen, dass die Signalerzeugungsvorrichtung die einzelnen Kapazitäten nacheinander mit der Wechselspannung beaufschlagt und/oder dass die Auswertevorrichtung die Impedanzverläufe in Bezug auf die einzelnen Kapazitäten nacheinander misst. Es ist aber im Falle einer gemeinsamen oder gleichzeitigen Überprüfung keine mechanische Manipulation der zu überprüfenden medizinischen Elektroden erforderlich.

Bevorzugt ist vorgesehen, dass die Beaufschlagung wenigstens einer der vorhandenen Kapazitäten mit der Wechselspannung so erzeugbar ist, dass die Signalerzeugungsvorrichtung die erste und/oder die zweite zu überprüfende medizinische Elektrode beaufschlagt.

Die Prüfvorrichtung selbst kann bevorzugt so ausgebildet sein, dass die Prüfvorrichtung zumindest einen Aufnahmebereich - vorzugsweise in Form einer ebenen Auflagefläche - aufweist, der zur Aufnahme wenigstens einer - vorzugsweise mehrerer - zu überprüfenden medizinischen Elektrode(n) ausgebildet ist, wobei die Messelektrode(n) im Aufnahmebereich angeordnet sind. Man kann damit die zu prüfende Elektrode oder das zu prüfende Elektrodenpaar einfach auf den Aufnahmebereich legen, worauf dann die kapazitive Messung erfolgt.

Zum Anschluss der Elektroden an die Signalerzeugungsvorrichtung kann vorteilhaft vorgesehen sein, dass vorzugsweise im Bereich eines Aufnahmebereiches für mindestens eine zu prüfende Elektrode - wenigstens ein - vorzugsweise mit der Signalerzeugungsvorrichtung verbundenes - elektrisches Anschlusselement angeordnet, mit dem mindestens ein Anschlussstecker eines zur medizinischen Elektrode führenden Anschlusskabels lösbar verbindbar ist.

Mit einer derartigen Ausbildung wird einfach der Anschlussstecker des Anschlusskabels der zu prüfenden Elektrode in das feststehende Anschlusselement eingesteckt und damit elektrisch mit der Signalerzeugungsvorrichtung verbunden.

Insgesamt kann mit der erfindungsgemäßen Prüfvorrichtung nicht nur die medizinische Elektrode selbst überprüft werden, sondern auch deren Anschlusskabel bzw. die leitende Verbindung desselben.

Bei zwei oder mehreren Elektroden kann auch die Richtigkeit (Polarität) der Anschlusskabel in allfälligen Anschlusssteckern überprüft werden. Das ist vor allem dann günstig, wenn bei zwei Elektroden zwei Anschlusskabel zu einem Doppel-Anschlussstecker führen, der mechanische Mittel aufweist, um beispielsweise in ein EKG-Gerät nur lagerichtig, also polaritätsrichtig eingesetzt zu werden. Wären die Anschlusskabel der beiden Elektroden vertauscht, so wäre die Polarität falsch und die erfindungsgemäße Prüfeinrichtung würde diesen Fehler detektieren. Diese falsch angeschlossene Doppelelektrode wird dann ausgeschieden und kommt nicht zur Anbringung auf der Haut eines insbesondere menschlichen Patienten.

Ein Aspekt der Erfindung besteht in der Verwendung einer von der Haut des Patienten gesonderten Prüfeinrichtung zum Überprüfen mindestens einer medizinischen Elektrode, bevor diese auf die Haut des Patienten appliziert, insbesondere aufgeklebt wird.

Es gibt bereits kapazitive Prüfeinrichtungen, die Übergangsimpedanz einer medizinischen Elektrode zur Haut des Patienten im aufgeklebten Zustand der Elektrode prüfen. Hier handelt es sich aber nicht um eine Prüfvorrichtung für die Elektroden selbst, sondern um eine Prüfvorrichtung für das richtige Aufkleben auf der Haut. Gemäß der erfindungsgemäßen Verwendung kommen schadhafte oder in der Polarität falsche Elektroden bzw. Elektrodenpaare überhaupt nicht zur Anwendung und können schon vorher ausgeschieden werden.

Wie bereits erwähnt, wird mit der erfindungsgemäßen Prüfvorrichtung in einer bevorzugten Verwendung nicht nur die Elektrode selbst überprüft, sondern auch deren Anschlusskabel bzw. gegebenenfalls vorhandene Anschlussstecker. Die Gesamtheit Elektrode, Anschlusskabel, Anschlussstecker, wie sie als Einheit, insbesondere in einer Verpackung, verkauft wird, ist damit schon vor der Anbringung auf der Haut des Patienten kapazitiv geprüft.

Ein Ausführungsbeispiel der Erfindung wird anhand der Figuren diskutiert. Es zeigen:
- Fig. 1: einen Schnitt durch die Explosionsdarstellung der Fig. 2
- Fig. 2: eine Explosionsdarstellung eines ersten Ausführungsbeispiels einer erfindungsgemäßen Prüfvorrichtung
- Fig. 3: eine schematische Darstellung einer erfindungsgemäßen Prüfvorrichtung
- Fig. 4: einen Schnitt durch die Explosionsdarstellung der Fig. 5
- Fig. 5: eine Explosionsdarstellung eines zweiten Ausführungsbeispiels einer erfindungsgemäßen Prüfvorrichtung
- Fig. 6: ein Prinzipschaltbild der Prüfvorrichtung

Es werden im Folgenden die Bezugszeichen C₁₁, C₁₂, C₂₁, C₂₂ für die erste bis vierte Kapazität verwendet, welche zwischen verschiedenen zu überprüfenden medizinischen Elektroden und verschiedenen Messelektroden vorliegen können. Die Verwendung unterschiedlicher Kurzbezeichnungen schließt natürlich nicht aus, dass die numerischen Werte der unterschiedlichen Kapazitäten gleich sein können.

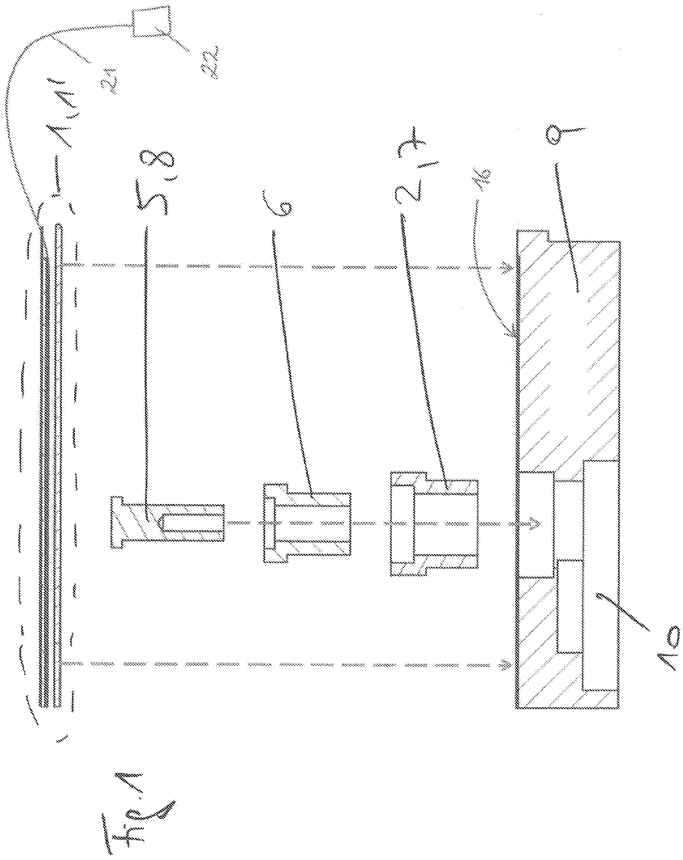

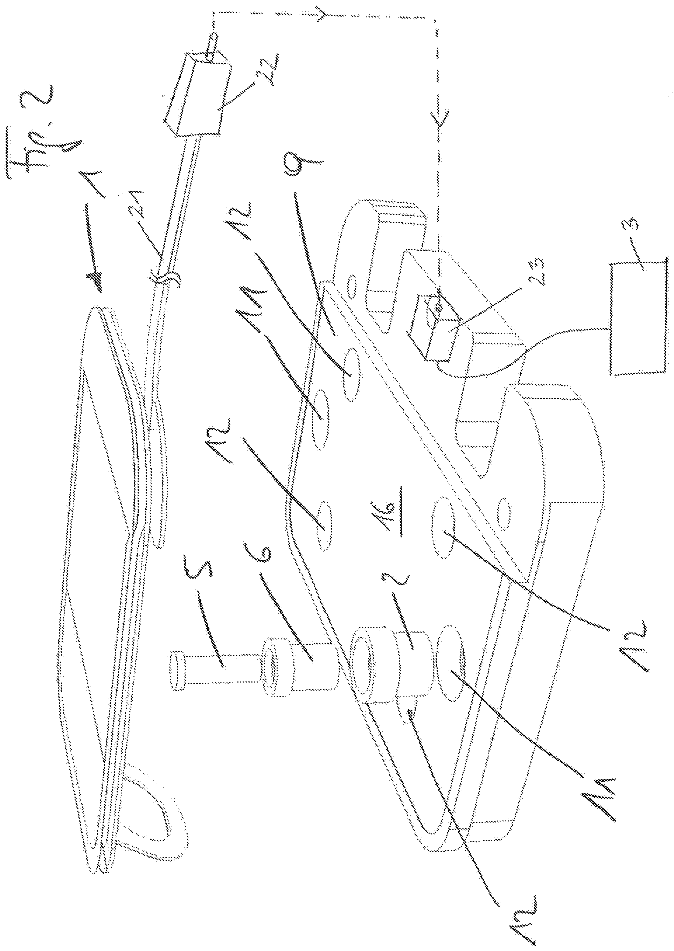

Das in den Fig. 1 und 2 gezeigte erste Ausführungsbeispiel einer erfindungsgemäßen Prüfvorrichtung eignet sich zur gemeinsamen Prüfung zweier medizinischer Elektroden (einer ersten und einer zweiten medizinischen Elektrode 1, 1') und zwar jeweils mit zwei Messelektroden (in Bezug auf die erste zu prüfende medizinische Elektrode 1 sind das die erste und die zweite Messelektrode 2, 5 und in Bezug auf die zweite zu prüfende medizinische Elektrode 1' sind das die dritte und die vierte Messelektrode 7, 8). Während der Prüfung liegen die vier Kapazitäten C₁₁, C₁₂, C₂₁, C₂₂ vor.

Die Prüfvorrichtung weist einen Aufnahmebereich 16 auf, welcher hier zur gleichzeitigen Aufnahme von zwei zu prüfenden medizinischen Elektroden 1, 1' ausgebildet ist. Die zu prüfenden medizinischen Elektroden 1, 1' sind so im Aufnahmebereich 16 anzuordnen, dass ihre eigentlichen Elektrodenflächen (Leitflächen) vom Aufnahmebereich 16 weg zeigen. Die Rückseiten der zu prüfenden medizinischen Elektroden 1, 1' werden für die Prüfung über Vakuum (zum Anlegen desselben dienen die Öffnungen 12) fixiert. So kommt es zu keinem unmittelbaren elektrischen Kontakt zwischen den Leitflächen der zu prüfenden medizinischen Elektroden 1, 1' und der Messelektroden 2, 5, 7, 8. Will man die zu prüfenden medizinischen Elektroden 1, 1' mit ihren Vorderseiten im Aufnahmebereich 16 anordnen, müssten gesonderte Isolatoren zwischen den Leitflächen und den Messelektroden angeordnet werden.

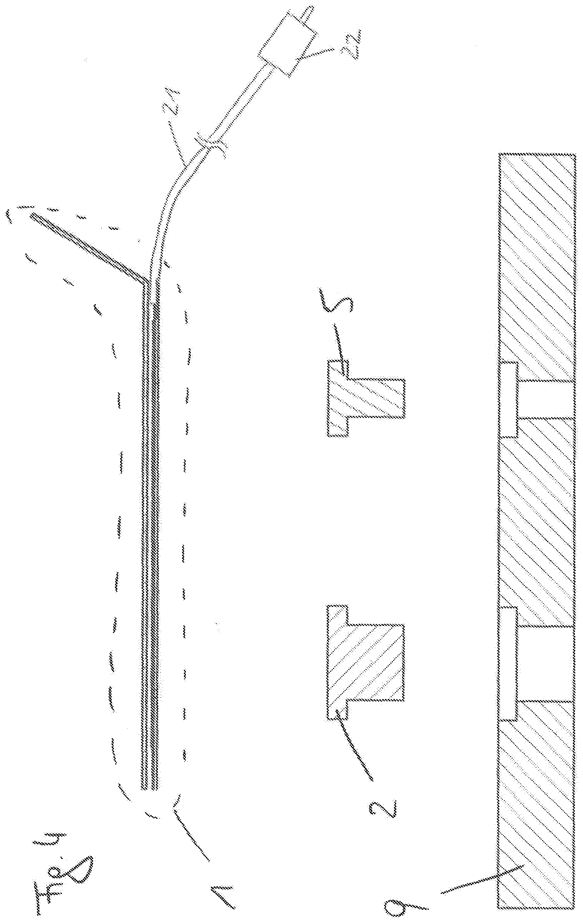

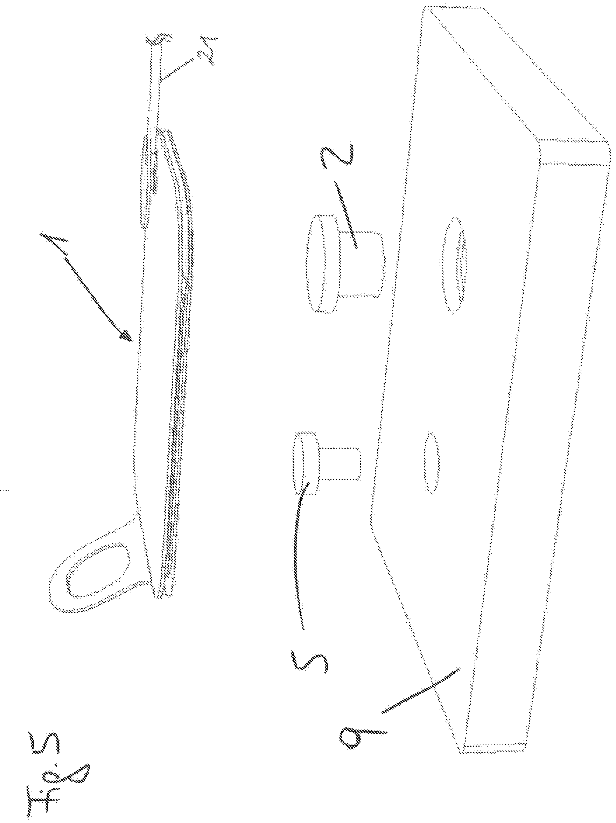

Das in den Fig. 4 und 5 gezeigte zweite Ausführungsbeispiel einer erfindungsgemäßen Prüfvorrichtung eignet sich Prüfung einer medizinischen Elektrode 1 und weist zwei Messelektroden 2, 5 auf. Diese sind nebeneinander angeordnet, könnten aber auch so ausgebildet sein wie im Ausführungsbeispiel der Fig. 1 und 2. Während der Prüfung liegen die zwei Kapazitäten C₁₁, C₁₂ vor. Der Träger 9 kann so wie im Ausführungsbeispiel der Fig. 1 und 2 ausgebildet sein.

Bei dem in Fig. 2 dargestellten Ausführungsbeispiel sieht man das Anschlusskabel 21 für die medizinische Elektrode 1 an dessen freien Ende ein Anschlussstecker 22 vorgesehen ist. Dieser ist lediglich schematisch dargestellt.

Neben dem Aufnahmebereich 16 für die zu überprüfende medizinische Elektrode ist ein Anschlusselement 23 angeordnet, in das der Anschlussstecker 22 zur Überprüfung eingesteckt werden kann. Die Signalerzeugungsvorrichtung kann dann über das Anschlusselement 23, den eingesteckten Anschlussstecker 22 und das Anschlusskabel 21 die medizinische Elektrode, insbesondere mit einer Wechselspannung beaufschlagen.

Durch diese Prüfvorrichtung wird nicht nur die Elektrode 1 selbst, sondern auch das Anschlusskabel und der Anschlussstecker 22 auf Funktionstüchtigkeit, insbesondere elektrische Leitfähigkeit überprüft.

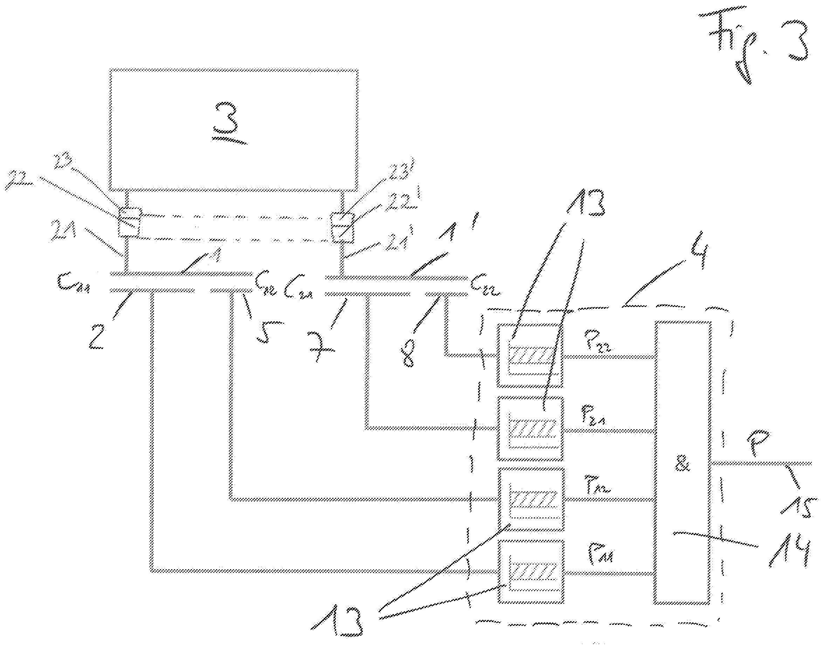

Fig. 3 zeigt sehr schematisch den Aufbau einer erfindungsgemäßen Prüfvorrichtung nach dem ersten Ausführungsbeispiel mit:
- einer ersten und einer zweiten Messelektrode 2, 5, die so relativ zu der ersten zu überprüfenden medizinischen Elektrode 1 anordenbar sind, dass die erste Messelektrode 2 und die erste zu überprüfende medizinische Elektrode 1 eine erste Kapazität C₁₁ bilden und die zweite Messelektrode 5 und die erste zu überprüfende medizinische Elektrode 1 eine zweite Kapazität C₁₂ bilden
- einer dritten und einer vierten Messelektrode 7, 8, die so relativ zu der zweiten zu überprüfenden medizinischen Elektrode 1' anordenbar sind, dass die dritte Messelektrode 7 und die zweite zu überprüfende medizinische Elektrode 1' eine dritte Kapazität C₂₁ bilden und die vierte Messelektrode 8 und die zweite zu überprüfende medizinische Elektrode 1' eine vierte Kapazität C₂₂ bilden
- einer Signalerzeugungsvorrichtung 3, über welche eine Wechselspannung erzeugbar ist, mittels welcher die erste bis vierte Kapazität C₁₁, C₁₂, C₂₁, C₂₂ beaufschlagbar ist
- einer Auswertevorrichtung 4, welche dazu ausgebildet ist, aus einem gemessenen Impedanzverlauf I einer in Reaktion auf die erste bis vierte Kapazität C₁₁, C₁₂, C₂₁, C₂₂ hervor gerufenen Impedanz ein erstes bis viertes Prüfergebnis P₁₁, P₁₂, P₂₁, P₂₂, in Bezug auf die erste bis vierte Kapazität C₁₁, C₁₂, C₂₁, C₂₂ zu bestimmen und ein gesamtes Prüfergebnis P in Bezug auf die erste bis vierte Kapazität C₁₁, C₁₂, C₂₁, C₂₂ zu bestimmen.

Bei dem in Fig. 3 dargestellten Ausführungsbeispiel können ebenfalls die Anschlusskabel 21, 21' sowie deren Anschlussstecker 22, 22' überprüft werden, wobei die Anschlussstecker 22, 22' in Anschlusselemente 23, 23' einsteckbar sind.

Wie durch strichlierte Linien angedeutete ist, können die Anschlussstecker 22, 22' zu einem Doppel-Anschlussstecker verbunden sein, der zwei elektrische Anschlüsse aufweist. Dasselbe gilt für die Anschlusselemente 23, 23'. Diese können ebenfalls zu einem Doppel-Anschlusselement verbunden sein.

Es können dann bevorzugt an sich bekannte mechanische Ausrichtungsmittel vorgesehen sein, die sicherstellen, dass der Doppel-Anschlussstecker 22, 22' nicht verkehrt in das Doppel-Anschlusselement 23, 23' eingesteckt wird. Solche Ausrichtungsmittel sind dem Fachmann bekannt, beispielsweise können verschieden große Anschlusszapfen oder asymmetrische Vorsprünge oder Nuten am Stecker verwendet werden, um ein verkehrtes Einstecken zu vermeiden. Wenn der Doppel-Anschlussstecker 22, 22' einmal lagerichtig in das Doppel-Anschlusselement 23, 23' eingesteckt ist, dann kann die erfindungsgemäße Prüfvorrichtung feststellen, ob das Anschlusskabel aus dem Anschlussstecker 22 wirklich zu Elektrode 1 und nicht (falsch) zur Elektrode 1' führt. Damit kann die Polaritätsrichtigkeit der Doppelelektrode 1, 1' überprüft werden, abgesehen von der Leitfähigkeit der Anschlusskabel und dem richtigen Elektrodenaufbau an sich.

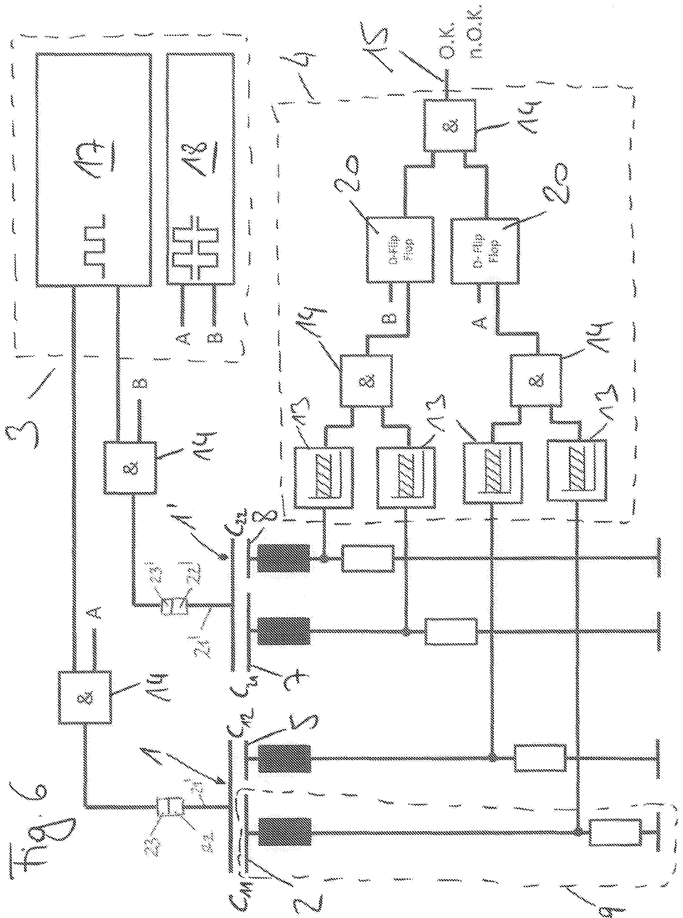

Eine detaillierte Darstellung zur Fig. 3 zeigt die Fig. 6. Zu erkennen ist, dass die Signalerzeugungsvorrichtung 3 einen Frequenzgenerator 17 und einen Taktgeber 18 umfasst. Der Frequenzgenerator 17 beaufschlagt die beiden zu prüfenden medizinischen Elektroden 1, 1' mit einer Wechselspannung. Der Taktgeber 18 weist einen Ausgang A und einen Ausgang B auf. Der Ausgang A ist der ersten zu prüfenden medizinischen Elektrode 1 zugeordnet und ist über ein UND-Glied 14 mit einem der ersten zu prüfenden medizinischen Elektrode 1 zugeordneten Ausgang des Frequenzgenerators 17 verknüpft. Der Ausgang B ist der zweiten zu prüfenden medizinischen Elektrode 1' zugeordnet und ist über ein UND-Glied 14 mit einem der zweiten zu prüfenden medizinischen Elektrode 1' zugeordneten Ausgang des Frequenzgenerators 17 verknüpft. So wird sichergestellt, dass die beiden zu prüfenden medizinischen Elektroden 1, 1' nicht gleichzeitig mit Wechselspannung beaufschlagt werden.

Die Ausgänge A, B des Taktgebers 18 sind des Weiteren jeweils mit einem Flip Flop 20 der Auswertevorrichtung 4 verknüpft. So lässt sich zuordnen, von welcher der beiden zu prüfenden medizinischen Elektroden 1, 1' gerade Prüfsignale eintreffen.

Jede der vier Kapazitäten C₁₁, C₁₂, C₂₁, C₂₂ ist Teil eines Schwingkreises 19 mit einer Induktivität und einem elektrischen Widerstand (der Übersichtlichkeit halber ist nur einer der Schwingkreise mit dem Bezugszeichen 19 versehen).

Der sich durch die angelegte Wechselspannung in jedem Schwingkreis 19 ergebende Impedanzverlauf I wird einer Analysevorrichtung 13 der Auswertevorrichtung 4 zugeführt, die überprüft, ob sich der Impedanzverlauf I in einem vorgegebenen Fenster befindet. Ist dies der Fall, gibt die jeweilige Analysevorrichtung 13 ein positives Prüfergebnis P₁₁, P₁₂, P₂₁, P₂₂ aus. Über ein UND-Glied 14 wird für jede der zu prüfenden medizinischen Elektroden 1, 1' ein Prüfergebnis gebildet. Über ein weiteres UND-Glied 14 wird ein gesamtes Prüfergebnis P für beide zu prüfenden medizinischen Elektroden 1, 1' gebildet und über den Ausgang 15 ausgegeben.

Die erfindungsgemäße Prüfvorrichtung eignet sich insbesondere zur Verwendung als von der Haut des Patienten gesonderte Prüfvorrichtung zum Überprüfen mindestens einer medizinischen Elektrode vor deren Applikation auf die Haut des Patienten. Nicht für in Ordnung befundene medizinische Elektroden können dann bereits vorab ausgeschieden werden und kommen überhaupt nicht zur Anwendung am Patienten. Mit der erfindungsgemäßen Prüfvorrichtung lässt sich nicht nur die Elektrode selbst, sondern auch deren Anschlusskabel und deren Anschlussstecker überprüfen, insbesondere auch bei einer Doppelelektrode mit einem Doppel-Anschlussstecker, denn Polaritätsrichtigkeit überprüft werden kann.

Die Überprüfung der medizinischen Elektrode samt Anschlusskabeln und Anschlusssteckern kann vor deren Verpackung in eine Verpackungshülle geschehen, sodass ausgeschiedene Elektroden von vorne herein nicht verpackt werden. Es ist aber auch denkbar, die Elektroden innerhalb einer (Teil)verpackung zu überprüfen, da die kapazitive Prüfung prinzipiell durch die Verpackungshülle hindurch geschehen kann.

### Bezugszeichenliste:

- 1: erste zu überprüfende medizinische Elektrode
- 1': zweite zu überprüfende medizinische Elektrode
- 2: erste Messelektrode
- 3: Signalerzeugungsvorrichtung
- 4: Auswertevorrichtung
- 5: zweite Messelektrode
- 6: Isolator
- 7: dritte Messelektrode
- 8: vierte Messelektrode
- 9: Träger
- 10: Raum für elektrische Kontaktierung der Messelektroden
- 11: Aufnahmeöffnung für Messelektroden
- 12: Öffnungen zum Beaufschlagen mit Vakuum
- 13: Analysevorrichtung
- 14: UND-Glied
- 15: Ausgang
- 16: Aufnahmebereich für medizinische Elektrode(n)
- 17: Frequenzgenerator
- 18: Taktgeber
- 19: Schwingkreis
- 20: Flip Flop
- 21, 21': Anschlusskabel
- 22, 22': Anschlussstecker
- 23, 23': Anschlusselement

- C₁₁: erste Kapazität (zwischen einer ersten zu überprüfenden medizinischen Elektrode und einer ersten Messelektrode)
- C₁₂: zweite Kapazität (zwischen einer ersten zu überprüfenden medizinischen Elektrode und einer zweiten Messelektrode)
- C₂₁: dritte Kapazität (zwischen einer zweiten zu überprüfenden medizinischen Elektrode und einer dritten Messelektrode)
- C₂₂: vierte Kapazität (zwischen einer zweiten zu überprüfenden medizinischen Elektrode und einer vierten Messelektrode)

- I: Impedanzverlauf

- P₁₁: erstes Prüfergebnis (bzgl. erster zu überprüfender Elektrode und erster Messelektrode)
- P₁₂: zweites Prüfergebnis (bzgl. erster zu überprüfender Elektrode und zweiter Messelektrode)
- P₂₁: erstes Prüfergebnis (bzgl. zweiter zu überprüfender Elektrode und erster Messelektrode)
- P₂₂: zweites Prüfergebnis (bzgl. zweiter zu überprüfender Elektrode und zweiter Messelektrode)
- P: gesamtes Prüfergebnis

## Patentansprüche

1. Prüfvorrichtung zum Überprüfen wenigstens einer ersten medizinischen Elektrode (1), **dadurch gekennzeichnet, dass** die Prüfvorrichtung aufweist:
- zumindest eine erste Messelektrode (2), die so relativ zu der ersten zu überprüfenden medizinischen Elektrode (1) anordenbar ist, dass die zumindest eine erste Messelektrode (2) und die erste zu überprüfende medizinische Elektrode (1) eine erste Kapazität (C₁₁) bilden
- eine Signalerzeugungsvorrichtung (3), über welche eine Wechselspannung erzeugbar ist, mittels welcher die erste Kapazität (C₁₁) beaufschlagbar ist
- eine Auswertevorrichtung (4), welche dazu ausgebildet ist, aus einem gemessenen Impedanzverlauf (I) einer in Reaktion auf die erste Kapazität (C₁₁) hervor gerufenen Impedanz zumindest ein erstes Prüfergebnis (P₁₁) in Bezug auf die erste Kapazität (C₁₁) zu bestimmen,
wobei die Messelektrode (2, 5, 7, 8) einerseits und die mit der Haut des Patienten in Kontakt zu bringenden medizinischen Elektroden (1, 1') andererseits verschiedene Elektroden sind.

2. Prüfvorrichtung nach Anspruch 1, wobei
- die Prüfvorrichtung weiters zumindest eine zweite Messelektrode (5) aufweist, die so relativ zu der ersten zu überprüfenden medizinischen Elektrode (1) anordenbar ist, dass die zumindest eine zweite Messelektrode (5) und die erste zu überprüfende medizinische Elektrode (1) eine zweite Kapazität (C₁₂) bilden
- die Auswertevorrichtung (4), dazu ausgebildet ist, aus einem gemessenen Impedanzverlauf (I) einer in Reaktion auf eine durch die Signalerzeugungsvorrichtung (3) erzeugte Wechselspannung aufgrund der zweiten Kapazität (C₁₂) hervor gerufenen Impedanz zumindest ein zweites Prüfergebnis (P₁₂) in Bezug auf die zweite Kapazität (C₁₂) zu bestimmen

3. Prüfvorrichtung nach dem vorangehenden Anspruch, wobei die Flächen der zumindest einen ersten und der zumindest einen zweiten Messelektrode (2, 5) unterschiedlich groß sind.

4. Prüfvorrichtung nach dem vorangehenden Anspruch, wobei die zumindest eine erste Messelektrode (2) als Kreisring ausgebildet ist und die zumindest eine zweite Messelektrode (5) innerhalb des Kreisrings angeordnet und durch einen Isolator (6) vom Kreisring elektrisch getrennt ist.

5. Prüfvorrichtung nach wenigstens einem der vorangehenden Ansprüche, wobei die Prüfvorrichtung zur gemeinsamen Überprüfung wenigstens einer ersten medizinischen Elektrode (1) und einer zweiten medizinischen Elektrode (1') ausgebildet ist und weiters aufweist:
- zumindest eine dritte Messelektrode (7), die so relativ zu der zweiten zu überprüfenden medizinischen Elektrode (1') anordenbar ist, dass die zumindest eine dritte Messelektrode (7) und die zweite zu überprüfende medizinische Elektrode (1') eine dritte Kapazität (C₂₁) bilden, wobei
- die Signalerzeugungsvorrichtung (3) dazu ausgebildet ist, über eine Wechselspannung zu erzeugen, mittels welcher die dritte Kapazität (C₂₁) beaufschlagbar ist
- die Auswertevorrichtung (4), die dazu ausgebildet ist, aus einem gemessenen Impedanzverlauf (I) einer in Reaktion auf die durch die Signalerzeugungsvorrichtung (3) erzeugte Wechselspannung aufgrund der dritten Kapazität (C₂₁) hervor gerufenen Impedanz zumindest ein erstes Prüfergebnis (P₂₁) in Bezug auf die dritte Kapazität (C₂₁) zu bestimmen

6. Prüfvorrichtung nach dem vorangehenden Anspruch, wobei
- die Prüfvorrichtung weiters zumindest eine vierte Messelektrode (8) aufweist, die so relativ zu der zweiten zu überprüfenden medizinischen Elektrode (1') anordenbar ist, dass die zumindest eine vierte Messelektrode (8) und die zweite zu überprüfende medizinische Elektrode (1') eine vierte Kapazität (C₂₂) bilden
- die Auswertevorrichtung (4), dazu ausgebildet ist, aus einem gemessenen Impedanzverlauf (I) einer in Reaktion auf eine durch die Signalerzeugungsvorrichtung (3) erzeugte Wechselspannung aufgrund der vierten Kapazität (C₂₂) hervor gerufenen Impedanz zumindest ein zweites Prüfergebnis (P₂₂) in Bezug auf die vierte Kapazität (C₂₂) zu bestimmen

7. Prüfvorrichtung nach dem vorangehenden Anspruch, wobei die Flächen der zumindest einen dritten und der zumindest einen vierten Messelektrode (7, 8) unterschiedlich groß sind.

8. Prüfvorrichtung nach dem vorangehenden Anspruch, wobei die zumindest eine dritte Messelektrode (7) als Kreisring ausgebildet ist und die zumindest eine vierte Messelektrode (8) innerhalb des Kreisrings angeordnet und durch einen Isolator (6) vom Kreisring elektrisch getrennt ist.

9. Prüfvorrichtung nach wenigstens einem der vorangehenden Ansprüche, wobei die Beaufschlagung wenigstens einer der vorhandenen Kapazitäten (C₁₁, C₁₂, C₂₁, C₂₂) mit der Wechselspannung so erzeugbar ist, dass die Signalerzeugungsvorrichtung (3) die erste und/oder die zweite zu überprüfende medizinische Elektrode (1, 1') beaufschlagt.

10. Prüfvorrichtung nach dem vorangehenden Anspruch, wobei die Auswertevorrichtung (4) mit der ersten und/oder der zweiten und/oder der dritten und/oder der vierten Messelektrode (2, 5, 7, 8) verbunden ist.

11. Prüfvorrichtung nach wenigstens einem der vorangehenden Ansprüche, wobei die Auswertevorrichtung (4) wenigstens eine Analysevorrichtung (13) aufweist, welche überprüft, ob der gemessene Impedanzverlauf (I) einer zugehörigen Kapazität (C₁₁, C₁₂, C₂₁, C₂₂) innerhalb eines vorgegebenen Prüffensters liegt und - falls dies der Fall ist - ein der jeweiligen Kapazität (C₁₁, C₁₂, C₂₁, C₂₂) entsprechendes positives Prüfergebnis (P₁₁, P₁₂, P₂₁, P₂₂) abgibt.

12. Prüfvorrichtung nach dem vorangehenden Anspruch, wobei die Auswertevorrichtung (4) ein UND-Glied (14) aufweist, welches nur bei Vorliegen eines positiven Prüfergebnisses (P₁₁, P₁₂, P₂₁, P₂₂) für alle gemessenen Kapazitäten (C₁₁, C₁₂, C₂₁, C₂₂) ein positives Gesamtprüfergebnis (P) für die gemessene Elektrode (1, 1') oder die Gesamtheit aller gemessenen Elektroden (1, 1') abgibt.

13. Prüfvorrichtung nach wenigstens einem der vorangehenden Ansprüche, wobei die Prüfvorrichtung zumindest einen Aufnahmebereich (16) - vorzugsweise in Form einer ebenen Auflagefläche - aufweist, der zur Aufnahme wenigstens einer - vorzugsweise mehrerer - zu überprüfenden medizinischen Elektrode(n) (1, 1') ausgebildet ist, wobei die Messelektrode(n) (2, 5, 7, 8) im Aufnahmebereich (16) angeordnet sind.

14. Prüfvorrichtung nach dem vorangehenden Anspruch, wobei im Aufnahmebereich (16) zumindest eine Öffnung (12) zum Beaufschlagen der zu prüfenden medizinischen Elektrode(n) (1, 1') mit Vakuum angeordnet ist.

15. Prüfvorrichtung nach einem der vorangehenden Ansprüche, wobei - vorzugsweise im Bereich eines Aufnahmebereiches (16) für mindestens eine zu prüfende Elektrode - wenigstens ein - vorzugsweise mit der Signalerzeugungsvorrichtung (3) verbundenes - elektrisches Anschlusselement (23) angeordnet, mit dem mindestens ein Anschlussstecker eines zur medizinischen Elektrode führenden Anschlusskabels (21, 21') lösbar verbindbar ist.

16. Verwendung einer von der Haut des Patienten gesonderten Prüfvorrichtung nach einem der Ansprüche 1 bis 15 zum Überprüfen mindestens einer medizinischen Elektrode (1, 1') vor deren Applikation auf die Haut des Patienten.

17. Verwendung einer Prüfvorrichtung nach einem der Ansprüche 1 bis 15 zum Überprüfen mindestens einer medizinischen Elektrode (1, 1') samt deren Anschlusskabel(n) (21, 21') und gegebenenfalls vorhandenen Anschlusssteckern, wobei die Wechselspannung aus der Signalerzeugungseinrichtung (3) über das (die) Anschlusskabel (21, 21') und gegebenenfalls den (die) Anschlussstecker (22, 22') an mindestens eine medizinische Elektrode (1, 1') angelegt wird.

18. Verwendung einer Prüfvorrichtung nach einem der Ansprüche 1 bis 15 zum Überprüfen einer medizinischen Elektrode, die sich in einer geschlossenen Verpackung befindet.

## Claims

1. Testing device for checking at least one first medical electrode (1), **characterised in that** the testing device has:
- at least one first measuring electrode (2), which can be arranged relative to the first medical electrode (1) to be checked, **in that** the at least one first measuring electrode (2) and the first medical electrode (1) to be checked form a first capacitance (C₁₁)
- a signal generating device (3), by way of which an alternating voltage can be generated, by means of which the first capacitance (C₁₁) can be acted upon
- an evaluation device (4), which is designed to determine at least one first test result (P₁₁) in relation to the first capacitance (C₁₁) from a measured impedance curve (I) of an impedance caused in response to the first capacitance (C₁₁),
wherein the measuring electrode (2, 5, 7, 8) on the one hand and the medical electrodes (1, 1') to be brought into contact with the skin of the patient on the other hand are different electrodes.

2. Testing device according to claim 1, wherein
- the testing device further comprises at least one second measuring electrode (5), which can be arranged relative to the first medical electrode (1) to be checked, in that the at least one second measuring electrode (5) and the first medical electrode (1) to be checked form a second capacitance (C12)
- the evaluation device (4) is designed to determine at least one second test result (P12) in relation to the second capacitance (C12) from a measured impedance curve (I) of an impedance caused due to the second capacitance (C12) in response to an alternating voltage generated by the signal generating device (3).

3. Testing device according to the preceding claim, wherein the surfaces of the at least one first and the at least one second measuring electrode (2, 5) are of different sizes.

4. Testing device according to the preceding claim, wherein the at least one first measuring electrode (2) is designed as a circular ring and the at least one second measuring electrode (5) is arranged within the circular ring and is electrically isolated from the circular ring by an insulator (6).

5. Testing device according to at least one of the preceding claims, wherein the testing device is designed to jointly check at least one first medical electrode (1) and a second medical electrode (1'), and further has:
- at least one third measuring electrode (7), which can be arranged relative to the second medical electrode (1') to be checked, in that the at least one third measuring electrode (7) and the second medical electrode (1') to be checked form a third capacitance (C21), wherein
- the signal generating device (3) is designed to generate an alternating voltage, by means of which the third capacitance (C21) can be acted upon
- the evaluation device (4), which is designed to determine at least one first test result (P21) in relation to the third capacitance (C21) from a measured impedance curve (I) of an impedance caused due to the third capacitance (C21) in response to the alternating voltage generated by the signal generating device (3).

6. Testing device according to the preceding claim, wherein
- the testing device further has at least one fourth measuring electrode (8), which can be arranged relative to the second medical electrode (1') to be checked, in that the at least one fourth measuring electrode (8) and the second medical electrode (1') to be checked form a fourth capacitance (C22),
- the evaluation device (4) is designed to determine at least one second test result (P22) in relation to the fourth capacitance (C22) from a measured impedance curve (I) of an impedance caused due to the fourth capacitance (C22) in response to an alternating voltage generated by the signal generating device (3).

7. Testing device according to the preceding claim, wherein the surfaces of the at least one third and the at least one fourth measuring electrode (7, 8) are of different sizes.

8. Testing device according to the preceding claim, wherein the at least one third measuring electrode (7) is designed as a circular ring and the at least one fourth measuring electrode (8) is arranged within the circular ring and is electrically isolated from the circular ring by an insulator (6).

9. Testing device according to at least one of the preceding claims, wherein the application of the alternating voltage to at least one of the existing capacitances (C₁₁, C₁₂, C₂₁, C₂₂) can be generated in such a way that the signal generating device (3) acts upon the first and/or the second medical electrode (1, 1') to be checked.

10. Testing device according to the preceding claim, wherein the evaluation device (4) is connected to the first and/or the second and/or the third and/or the fourth measuring electrode (2, 5, 7, 8).

11. Testing device according to at least one of the preceding claims, wherein the evaluation device (4) has at least one analysis device (13) which checks whether the measured impedance profile (I) of an associated capacitance (C₁₁, C₁₂, C₂₁, C₂₂) lies within a predetermined test window and, if this is the case, emits a positive test result (P₁₁, P₁₂, P₂₁, P₂₂) corresponding to the respective capacitance (C₁₁, C₁₂, C₂₁, C₂₂).

12. Testing device according to the preceding claim, wherein the evaluation device (4) has an AND element (14) which emits a positive overall test result (P) for the measured electrode (1, 1') or the totality of all the measured electrodes (1, 1') only when a positive test result (P₁₁, P₁₂, P₂₁, P₂₂) exists for all measured capacitances (C₁₁, C₁₂, C₂₁, C₂₂).

13. Testing device according to at least one of the preceding claims, wherein the testing device has at least one receiving region (16), preferably in the form of a planar support surface, designed to receive at least one, preferably a plurality of, medical electrode(s) (1, 1') to be checked, wherein the measuring electrode(s) (2, 5, 7, 8) is/are arranged in the receiving region (16).

14. Testing device according to the preceding claim, wherein at least one opening (12) is arranged in the receiving region (16) in order to apply a vacuum to the medical electrode(s) (1, 1') to be checked.

15. Testing device according to one of the preceding claims, wherein arranged preferably in the region of a receiving region (16) for at least one electrode to be checked is at least one electrical connection element (23), preferably connected to the signal generating device (3), to which element at least one connector plug of a connecting cable (21, 21') leading to the medical electrode can be detachably connected.

16. Use of a testing device, which is separate from the skin of the patient, according to one of claims 1 to 15 for checking at least one medical electrode (1, 1') before it is applied to the skin of the patient.

17. Use of a testing device according to one of claims 1 to 15 for testing at least one medical electrode (1, 1') together with its connecting cable(s) (21, 21') and optionally present connector plugs, wherein the alternating voltage from the signal generating device (3) is applied to at least one medical electrode (1, 1') via the connecting cable(s) (21, 21') and, optionally, the connector plug(s) (22, 22').

18. Use of a testing device according to one of claims 1 to 15 for checking a medical electrode located in a closed package.

## Revendications

1. Dispositif de contrôle pour le contrôle d'au moins une première électrode médicale (1), **caractérisé en ce que** le dispositif de contrôle comprend :
- au moins une première électrode de mesure (2), qui peut être disposée par rapport à la première électrode médicale (1) à contrôler de façon à ce que l'au moins une première électrode de mesure (2) et la première électrode médicale (1) à contrôler forment une première capacité (C₁₁)
- un dispositif de production de signaux (3) par l'intermédiaire duquel une tension alternative peut être générée, au moyen de laquelle la première capacité (C₁₁) peut être alimentée
- un dispositif d'analyse (4) qui est conçu pour déterminer, à partir d'un tracé d'impédance (I) mesuré d'une impédance générée en réaction à la première capacité (C₁₁), au moins un premier résultat de contrôle (P₁₁) concernant la première capacité (C₁₁),
dans lequel l'électrode de mesure (2, 5, 7, 8) d'une part et les électrodes médicales (1, 1') à mettre en contact avec la peau du patient d'autre part sont des électrodes différentes.

2. Dispositif de contrôle selon la revendication 1, dans lequel
- le dispositif de contrôle comprend en outre au moins une deuxième électrode de mesure (5) qui peut être disposée par rapport à la première électrode médicale (1) à contrôler de façon à ce que l'au moins une deuxième électrode de mesure (5) et la première électrode médicale (1) à contrôler forment une deuxième capacité (C₁₂)
- le dispositif d'analyse (4) est conçu pour déterminer, à partir d'un tracé d'impédance (I) mesuré d'une impédance générée sur la base de la deuxième capacité (C₁₂), en réaction à la tension alternative générée par le dispositif de production de signaux (3), un deuxième résultat de contrôle (P₁₂) concernant la deuxième capacité (C₁₂).

3. Dispositif de contrôle selon la revendication précédente, dans lequel les surfaces de l'au moins une première et de l'au moins une deuxième électrode de mesure (2, 5) sont de tailles différentes.

4. Dispositif de contrôle selon la revendication précédente, dans lequel l'au moins une première électrode de mesure (2) présente la forme d'un anneau et l'au moins une deuxième électrode de mesure (5) est disposée à l'intérieur de cet anneau et est électriquement isolée de l'anneau par un isolateur (6).

5. Dispositif de contrôle selon au moins l'une des revendications précédentes, dans lequel le dispositif de contrôle est conçu pour le contrôle commun d'au moins une première électrode médicale (1) et d'une deuxième électrode médicale (1') et comprend en outre :
- au moins une troisième électrode de mesure (7), qui peut être disposée par rapport à la deuxième électrode médicale (1') à contrôler de façon à ce que l'au moins une troisième électrode de mesure (7) et la deuxième électrode médicale (1') à contrôler forment une troisième capacité (C₂₁), dans lequel
- le dispositif de production de signaux (3) est conçu pour générer une tension alternative par l'intermédiaire de laquelle la troisième capacité (C₂₁) peut être alimentée
- le dispositif d'analyse (4), qui est conçu pour déterminer, à partir d'un tracé d'impédance (I) mesuré d'une impédance générée sur la base de la troisième capacité (C₂₁), en réaction à la tension alternative générée par le dispositif de production de signaux (3), au moins un premier résultat de contrôle (P₂₁) concernant la troisième capacité (C₂₁).

6. Dispositif de contrôle selon la revendication précédente, dans lequel
- le dispositif de contrôle comprend en outre au moins une quatrième électrode de mesure (8) qui peut être disposée par rapport à la deuxième électrode médicale (1') à contrôler de façon à ce que l'au moins une quatrième électrode de mesure (8) et la deuxième électrode médicale (1') à contrôler forment une quatrième capacité (C₂₂)
- le dispositif d'analyse (4) est conçu pour déterminer, à partir d'un tracé d'impédance (I) mesuré d'une impédance générée sur la base de la quatrième capacité (C₂₂), en réaction à la tension alternative générée par le dispositif de production de signaux (3), au moins un deuxième résultat de contrôle (P₂₂) concernant la quatrième capacité (C₂₂).

7. Dispositif de contrôle selon la revendication précédente, dans lequel les surfaces de l'au moins une troisième et de l'au moins une quatrième électrode de mesure (7, 8) sont de tailles différentes.

8. Dispositif de contrôle selon la revendication précédente, dans lequel l'au moins une troisième électrode de mesure (7) présente la forme d'un anneau et l'au moins une quatrième électrode de mesure (8) est disposée à l'intérieur de cet anneau et est électriquement isolée de l'anneau par un isolateur (6).

9. Dispositif de contrôle selon au moins l'une des revendications précédentes, dans lequel l'alimentation d'au moins une des capacités existantes (C₁₁, C₁₂, C₂₁, C₂₂) peut être générée avec la tension alternative de façon à ce que le dispositif de production de signaux (3) alimente la première et/ou la deuxième électrode médicale (1, 1') à contrôler.

10. Dispositif de contrôle selon la revendication précédente, dans lequel le dispositif d'analyse (4) est relié avec la première et/ou la deuxième et/ou la troisième et/ou la quatrième électrode de mesure (2, 5, 7, 8).

11. Dispositif de contrôle selon au moins l'une des revendications précédentes, dans lequel le dispositif d'analyse (4) comprend au moins un dispositif d'analyse (13) qui contrôle si le tracé d'impédance mesuré (I) d'une capacité (C₁₁, C₁₂, C₂₁, C₂₂) correspondante se trouve à l'intérieur de la plage de contrôle prédéterminée et, si c'est le cas, génère un résultat de contrôle positif (P₁₁, P₁₂, P₂₁, P₂₂) correspondant à la capacité (C₁₁, C₁₂, C₂₁, C₂₂) correspondante.

12. Dispositif de contrôle selon la revendication précédente, dans lequel le dispositif d'analyse (4) comprend un organe UND (14) qui ne génère un résultat de contrôle général positif (P) pour l'électrode (1, 1') mesurée ou l'ensemble de toutes les électrodes (1, 1') mesurées que lors de l'existence d'un résultat de contrôle positif (P₁₁, P₁₂, P₂₁, P₂₂) pour toutes les capacités (C₁₁, C₁₂, C₂₁, C₂₂) mesurées.

13. Dispositif de contrôle selon au moins l'une des revendications précédentes, dans lequel le dispositif de contrôle comprend au moine une partie de logement (16), de préférence sous la forme d'une surface d'appui plane, qui est conçue pour le logement d'au moins une, de préférence plusieurs, électrode(s) médicale(s) (1, 1'), dans lequel la/les électrode(s) de mesure (2, 5, 7, 8) est/sont disposée(s) dans la partie de logement (16).

14. Dispositif de contrôle selon la revendication précédente, dans lequel, dans la partie de logement (16), est disposée au moins une ouverture (12) pour l'alimentation de l'électrode / des électrodes médicale(s) (1, 1') avec du vide.

15. Dispositif de contrôle selon au moins l'une des revendications précédentes, dans lequel, de préférence au niveau d'une partie de logement (16) pour au moins une électrode à contrôler, est disposé au moins un élément de raccordement électrique (23), de préférence relié avec le dispositif de production de signaux (3), avec lequel au moins une prise de raccordement d'un câble de raccordement (21, 21') conduisant à l'électrode médicale peut être relié de manière amovible.

16. Utilisation d'un dispositif de contrôle séparé de la peau du patient selon l'une des revendications 1 à 15 pour le contrôle d'au moins une électrode médicale (1, 1') avant son application sur la peau du patient.

17. Utilisation d'un dispositif de contrôle selon l'une des revendications 1 à 15 pour le contrôle d'au moins une électrode médicale (1, 1') avec son/ses câble(s) de raccordement (21, 21'), dans lequel la tension alternative est appliquée, à partir du dispositif de production de signaux (3), par l'intermédiaire du/des câble(s) de raccordement (21, 21') et, le cas échéant, du/des prise(s) de raccordement (22, 22'), à au moins une électrode médicale (1, 1').

18. Utilisation d'un dispositif de contrôle selon l'une des revendications 1 à 15 pour le contrôle d'une électrode médicale qui se trouve dans un emballage fermé.
